# EUROPÄISCHE PATENTSCHRIFT

(11) **EP 2 654 815 B1**
(45) Veröffentlichungstag und Bekanntmachung des Hinweises auf die Patenterteilung: **11.03.2015**
(21) Anmeldenummer: 12700549.4
(22) Anmeldetag: 12.01.2012
(51) Int. Cl.: A61L 27/36

(54) **HERSTELLUNG EINES TRANSPLANTATS AUS TIERISCHER DERMIS MIT NATRIUMSULFIDLÖSUNG**
PRODUCING A TRANSPLANT FROM ANIMAL DERMIS USING SODIUM SULFIDE SOLUTION
PRODUCTION D'UN GREFFON À PARTIR DE DERME ANIMAL À L'AIDE UNE SOLUTION DE SULFURE DE SODIUM

(30) Priorität: 14.01.2011 DE 102011008604
(43) Veröffentlichungstag der Anmeldung: 30.10.2013
(73) Patentinhaber: Tutogen Medical GmbH, 91077 Neunkirchen am Brand (DE)
(72) Erfinder: WILHELMI, Arnd, 91301 Forchheim (DE); SCHREINER, Silke, 91054 Erlangen (DE)
(74) Vertreter: Manitz, Finsterwald & Partner GbR
(86) Internationale Anmeldenummer: PCT/EP2012/000124
(87) Internationale Veröffentlichungsnummer: WO 2012/095316

(56) Entgegenhaltungen:
- DE-B4- 10 196 234
- I. PRASERTSUNG ET AL.: "Development of acellular dermis from porcine skin using periodic pressurized technique", JOURNAL OF BIOMEDICAL MATERIAL RESEARCH PART B: APPLIED BIOMATERIALS, Bd. 85, Nr. 1, 12. September 2007 (2007-09-12), Seiten 210-219, XP002670156,

## Beschreibung

Die vorliegende Erfindung betrifft ein Verfahren zur Herstellung eines Transplantats aus tierischer Dermis, insbesondere aus porciner Dermis, sowie ein damit erhältliches Transplantat.

Gewebetransplantate werden für viele Anwendungen eingesetzt, beispielsweise bei zahnärztlichen Operationen zum Aufbau des Kieferknochens oder zur Weichgeweberegeneration, zum Auffüllen von Knochendefekten im Falle eines Tumors, bei Eingriffen zur Wiederherstellung von Sehnen und Bändern sowie bei der Korrektur angeborener Defekte. Darüber hinaus können sie auch bei Operationen an der Wirbelsäule, bei der Revision von Hüftprothesen oder bei der Behandlung von Bauchwanddefekten eingesetzt werden.

Grundsätzlich werden drei Arten von Gewebetransplantaten unterschieden, nämlich autogene Gewebetransplantate, bei denen Spender und Empfänger dieselbe Person sind, wobei das Transplantat üblicherweise an einer anderen Stelle als der Entnahmestelle eingesetzt wird, allogene Gewebetransplantate, bei denen Spender und Empfänger verschiedene Individuen einer Spezies sind sowie xenogene Gewebetransplantate, bei denen der Spender einer anderen Art, beispielsweise Schwein, angehört als der Empfänger, beispielsweise Mensch. Aufgrund der begrenzten Verfügbarkeit allogener und insbesondere autogener Gewebetransplantate sind xenogene Gewebetransplantate von besonderem Interesse. Dabei sind insbesondere Gewebetransplantate aus Schweinen, beispielsweise aus porciner Dermis, begehrt, weil diese physiologisch besser den Anforderungen an den menschlichen Körper genügen als beispielsweise Gewebetransplantate aus Primaten. Hinzu kommt die nahezu unbegrenzte Verfügbarkeit von porciner Haut.

Ein wesentlicher Aspekt bei der Herstellung von Gewebetransplantaten ist es, dass die native Struktur des Gewebes in dem fertigen Transplantat möglichst beibehalten wird, damit dieses beispielsweise im Hinblick auf die innere Oberfläche, die Handhabbarkeit und die Elastizität zumindest weitgehend die Eigenschaften des Nativgewebes aufweist. Allerdings ist es mit den bisher bekannten Verfahren nicht möglich, Transplantate aus tierischer Dermis und insbesondere aus porciner Dermis bzw. Schweinedermis unter ausreichender Beibehaltung der nativen Gewebestruktur herzustellen. Üblicherweise werden solche Gewebetransplantate nämlich durch ein Verfahren hergestellt, bei dem das Gewebe zunächst mit 1 M Natronlauge behandelt wird, um die Gewebestruktur aufzulockern, Borsten aus der Dermis aufzulösen und die Dermis zu enthaaren. Durch diesen Behandlungsschritt wird jedoch die dermale Kollagenmatrix stark geschädigt, so dass die native Struktur der Dermis beträchtlich beeinträchtigt wird.

Prasertsung et al. berichten in Journal of Biomedical Materials Research 2007, Seiten 210 bis 219 über das Ergebnis einer Studie, bei welcher die Effekte des Enzymtyps und der Druckbedingungen auf die Wirksamkeit der Zellentfernung bei der Herstellung eines Transplantats aus porciner Dermis untersucht worden sind. Dabei wurde Haut mit 20 prozentiger Natriumsulfidlösung behandelt, um die Haare von der Haut zu entfernen, bevor die enthaarte Haut nacheinander mit konzentrierter Kochsalzlösung und konzentrierter Glycerinlösung behandelt wurde.

Aufgabe der vorliegenden Erfindung ist es daher, ein Verfahren zur Herstellung eines Transplantats bereitzustellen, mit dem aus tierischer Dermis und insbesondere aus porciner Dermis einfach, schnell und kostengünstig ein Gewebetransplantat hergestellt werden kann, dessen Morphologie der nativen Struktur der Dermis zumindest weitgehend entspricht.

Erfindungsgemäß wird diese Aufgabe durch ein Verfahrens zur Herstellung eines Transplantats aus tierischer Dermis, insbesondere aus porciner Dermis, gelöst, welches die nachfolgenden Schritte umfasst:
a) Bereitstellen von tierischer Dermis,
b) Behandeln der tierischen Dermis mit einer wässrigen, alkalischen Natriumsulfid enthaltenden Lösung, welche 0,01 bis 1,5 Gew.-% Natriumsulfid enthält,
c) ein- oder mehrmaliges Behandeln der Dermis mit einer wässrigen Salzlösung,
d) ein- oder mehrmaliges Behandeln der Dermis mit einer wässrigen Wasserstoffperoxidlösung und
e) Dehydratisieren der Dermis.

Diese Lösung beruht auf der Erkenntnis, dass durch die Behandlung von tierischer Dermis mit einer Natriumsulfid enthaltenden Lösung, gleichermaßen wie durch die Behandlung mit einer vergleichsweise konzentrierten Natronlauge, eine Auflockerung der Gewebestruktur, eine Entfernung der Borsten aus der Dermismatrix und eine Enthaarung der Dermis erreicht werden kann, und zwar überraschenderweise so, dass im Unterschied zu der Behandlung mit einer vergleichsweise konzentrierten Natronlauge eine Schädigung der dermalen Kollagenmatrix der Dermis nahezu vollständig verhindert wird. Aus diesem Grund kann mit dem erfindungsgemäßen Verfahren aus einer tierischen Dermis einfach, schnell und kostengünstig ein steriles und zellfreies xenogenes Transplantat hergestellt werden, welches hinsichtlich seiner morphologischen Struktur zumindest weitgehend der nativen Gewebestruktur entspricht. Insbesondere kann bei dem erfindungsgemäßen Verfahren auf eine chemische Behandlung des Gewebes mit chemischen Agentien, welche eine Kreuzvernetzung von in dem Kollagen enthaltenen Proteinen unter Verlust der nativen Gewebestruktur verursachen, wie Formaldehyd, Glutaraldehyd oder dergleichen, verzichtet werden. Eine solche Kreuzvernetzung von Proteinen des Gewebes ist nachteilhaft, weil eine solche Kreuzvernetzung das Einwachsverhalten des Transplantats in dem Empfänger negativ beeinflusst. In manchen Fällen kann eine solche Proteinkreuzvernetzung sogar dazu führen, dass das

Transplantat in dem Empfänger überhaupt nicht einwächst, sondern eingekapselt wird, ohne vorher die vorgesehene Funktion der Gewebestabilisierung auszuüben.

Um bei der Durchführung des erfindungsgemäßen Verfahrens die Erzeugung von Schwefelwasserstoff zuverlässig zu verhindern, ist es erfindungsgemäß vorgesehen, in dem Verfahrensschritt b) eine wässrige alkalische, Natriumsulfid enthaltende Lösung einzusetzen. Grundsätzlich kann die bei dieser Ausführungsform eingesetzte alkalische, Natriumsulfid enthaltende Lösung jeden beliebigen pH-Wert von größer als 7 aufweisen, wobei insbesondere mit entsprechenden Lösungen, welche einen pH-Wert von wenigstens 10 und bevorzugt von wenigstens 12 aufweisen, gute Ergebnisse erhalten werden.

Vorzugsweise enthält die in dem Verfahrensschritt b) eingesetzten wässrige Lösung 0,75 bis 1,5 Gew.-% und höchst bevorzugt etwa 1 Gew.-% Natriumsulfid.

Um den vorstehend genannten bevorzugten alkalischen pH-Wert einzustellen, ist es gemäß einer bevorzugten Ausführungsform der vorliegenden Erfindung vorgesehen, dass in dem Verfahrensschritt b) eine wässrige Lösung eingesetzt wird, welche neben Natriumsulfid 0,001 bis 0,5 M Natriumhydroxid, bevorzugt 0,005 bis 0,4 M Natriumhydroxid, besonders bevorzugt 0,01 bis 0,3 M Natriumhydroxid, insbesondere bevorzugt 0,05 bis 0,2 M Natriumhydroxid, ganz besonders bevorzugt 0,075 bis 0,125 M Natriumhydroxid und höchst bevorzugt etwa 0,1 M Natriumhydroxid enthält.

Anstelle von Natriumhydroxid kann jede dem Fachmann zur Einstellung von pH-Werten bekannte Base eingesetzt werden, wie beispielsweise jedes Alkalimetallhydroxid oder Erdalkalimetallhydroxid, wie Kaliumhydroxid, Lithiumhydroxid, Magnesiumhydroxid, Calciumhydroxid, Ammoniak und dergleichen.

Grundsätzlich sollte die Natriumsulfidlösung in dem Verfahrensschritt b) in einer ausreichend hohen Menge und für eine ausreichend lange Zeit eingesetzt werden, damit die Dermis wirksam aufgelockert wird und die darin enthaltenen Borsten und Haare nahezu vollständig entfernt werden. Gute Ergebnisse werden diesbezüglich insbesondere erreicht, wenn die Dermis für 1 bis 48 Stunden, bevorzugt für 5 bis 36 Stunden, besonders bevorzugt für 12 bis 24 Stunden und ganz besonders bevorzugt für 14 bis 20 Stunden mit der wässrigen Natriumsulfid enthaltenden Lösung behandelt wird. Dabei wird die wässrige Natriumsulfid enthaltende Lösung vorzugsweise in dem 5- bis 15-fachen Volumen bezogen auf das Volumen der Dermis eingesetzt. Besonders bevorzugt wird die wässrige Natriumsulfid enthaltende Lösung in dem 6- bis 14-fachen Volumen, insbesondere bevorzugt in dem 7- bis 13-fachen Volumen, weiter bevorzugt in dem 8- bis 12-fachen Volumen, ganz besonders bevorzugt in dem 9- bis 11-fachen Volumen und höchst bevorzugt in einem etwa 10-fachen Volumen bezogen auf das Volumen der Dermis eingesetzt.

Prinzipiell kann die Behandlung mit der Natriumsulfid enthaltenden Lösung in dem Verfahrensschritt b) einmal oder mehrmals hintereinander durchgeführt werden, wobei die Dermis zwischen den einzelnen Behandlungsschritten mit der Natriumsulfid enthaltenden Lösung mit Wasser behandelt und/oder gespült wird. Allerdings hat es sich im Rahmen der vorliegenden Erfindung als bevorzugt herausgestellt, in dem Verfahrensschritt b) nur eine einmalige Behandlung der Dermis mit der Natriumsulfid enthaltenden Lösung vorzugsweise für die zuvor genannte Behandlungsdauer durchzuführen.

In Weiterbildung des Erfindungsgedankens wird es vorgeschlagen, die Behandlung mit der Natriumsulfidlösung gemäß dem Verfahrensschritt b) in einer rotierenden Trommel durchzuführen. Dadurch wird eine gleichmäßige und vollständige Inkubation der Dermis mit dem Natriumsulfid erreicht. Beispielsweise kann die rotierende Trommel horizontal gelagert und um ihre Achse rotiert werden. Zweckmäßigerweise sind an der Trommel Anschlüsse vorgesehen, über welche die Prozesslösungen in die Trommel zugeführt und aus der Trommel abgeführt werden können. Zudem können an der Trommel auch Anschlüsse vorgesehen sein, um in der Trommel je nach Bedarf einen Über- oder Unterdruck einzustellen. Zudem ist es bevorzugt, dass die Trommel doppelwandig ausgestaltet ist, damit der Innenraum der Trommel über in dem Hohlraum vorgesehene Anschlüsse temperiert werden kann. Schließlich ist es auch bevorzugt, die Trommel so auszugestalten, dass deren Drehgeschwindigkeit elektronisch regelbar ist.

Durch die Behandlung der Dermis mit einer wässrigen Salzlösung in dem Verfahrensschritt c) werden nach dem Verfahrensschritt b) noch vorhandene intakte Zellen durch osmotischen Stress zerstört. Dabei können alle zu diesem Zweck geeigneten Salze eingesetzt werden, wie insbesondere Alkalimetallhalogenide und Erdalkalimetallhalogenide. Aus Kostengründen sind für diesen Zweck insbesondere die Alkalimetallchloride und Erdalkalimetallchloride, wie insbesondere Natriumchlorid, geeignet.

Gute Ergebnisse werden in diesem Zusammenhang insbesondere erhalten, wenn die Dermis in dem Verfahrensschritt c) mit einer wässrigen, 1 bis 50 Gew.-%, bevorzugt 2 bis 20 Gew.-%, besonders bevorzugt 5 bis 15 Gew.-% und ganz besonders bevorzugt 10 Gew.-% Natriumchlorid enthaltenden Lösung behandelt wird. Diese Mengen sind auch bei der Verwendung anderer Alkalimetallhalogenide und Erdalkalimetallhalogenide, wie Kaliumchlorid, Lithiumchlorid, Magnesiumchlorid, Calciumchlorid und dergleichen, bevorzugt.

Gemäß einer weiteren bevorzugten Ausführungsform der vorliegenden Erfindung wird die Dermis in dem Verfahrensschritt c), jeweils unterbrochen durch eine Behandlung und/oder ein Spülen mit Wasser, mehrmals mit Salzlösung behandelt. Durch diese Wechselbadbehandlung Salzlösung/Wasser wird der Transport von zellulären Bestandteilen aus der Gewebestruktur durch Diffusionsvorgänge infolge wechselnder osmotischer Verhältnisse in dem Gewebe begünstigt und somit der Effekt der Salzbadbehandlung verbessert. Gute Ergebnisse werden dabei insbesondere erhalten, wenn die Dermis in dem Verfahrensschritt c) zwei- bis zehnmal, bevorzugt drei- bis fünfmal und besonders bevorzugt viermal mit einer wässrigen Salzlösung behandelt wird, wobei die Dermis zwischen den Behandlungsschritten mit Salzlösung jeweils mit Wasser behandelt wird.

Dabei beträgt die Dauer der einzelnen Behandlungsschritte in dem Verfahrensschritt c) vorzugsweise 1 bis 48 Stunden, wobei zumindest einer dieser Behandlungsschritte bevorzugt für 5 bis 36 Stunden, besonders bevorzugt für 12 bis 24 Stunden und höchst bevorzugt für 14 bis 20 Stunden, beispielsweise für 16 Stunden, durchgeführt wird.

Vorzugsweise wird auch der Verfahrensschritt c) in der zuvor für den Verfahrensschritt b) als bevorzugt beschriebenen rotierenden Trommel durchgeführt.

Um die in dem Gewebe vorhandenen Bakterien und Viren abzutöten, und, um das Gewebe zu bleichen, ist es erfindungsgemäß vorgesehen, den Verfahrensschritt d) durchzuführen, und zwar vorzugsweise unter Verwendung einer wässrigen Lösung, welche 1 bis 10 Gew.-% Wasserstoffperoxid, bevorzugt 2 bis 5 Gew.-% Wasserstoffperoxid und besonders bevorzugt etwa 3 Gew.-% Wasserstoffperoxid enthält.

Dabei wird die Dermis in dem Verfahrensschritt d) vorzugsweise mehrmals mit einer wässrigen Wasserstoffperoxidlösung behandelt, und zwar bevorzugt zwei- bis fünfmal und besonders bevorzugt dreimal, wobei die Dermis zwischen den Behandlungsschritten mit der Wasserstoffperoxidlösung jeweils mit Wasser behandelt und/oder gespült wird.

Gute Ergebnisse werden dabei insbesondere erhalten, wenn die Dauer der einzelnen Behandlungsschritte in dem Verfahrensschritt d) vorzugsweise 5 bis 60 Stunden, besonders bevorzugt 10 bis 36 Stunden und ganz besonders bevorzugt 20 bis 30 Stunden, beispielsweise 24 Stunden, beträgt.

Vorzugsweise wird auch der Verfahrensschritt d) in der zuvor für den Verfahrensschritt b) als bevorzugt beschriebenen rotierenden Trommel durchgeführt.

Um in dem Verfahrensschritt e) eine besonders effektive Dehydratisierung zu erreichen, welche eine anschließende Lagerung der Dermis im eingefrorenen Zustand ohne Veränderung ihrer Struktur ermöglicht, ist es gemäß einer weiteren bevorzugten Ausführungsform der vorliegenden Erfindung vorgesehen, die Dermis in dem Verfahrensschritt e) durch zwei- bis zehnmaliges, bevorzugt durch vier- bis achtmaliges und besonders bevorzugt durch sechsmaliges Behandeln mit einem organischen Lösungsmittel zu dehydratisieren. Dabei werden besonders gute Ergebnisse erhalten, wenn das organische Lösungsmittel aus der Gruppe ausgewählt wird, welche aus Aceton, Methanol, Ethanol und Isopropylalkohol besteht.

Gemäß einer besonders bevorzugten Ausführungsform der vorliegenden Erfindung wird in dem Verfahrensschritt e) Aceton als organisches Lösungsmittel eingesetzt, weil dieses nicht nur die Dermis dehydratisiert, sondern diese insbesondere auch entfettet und etwaig vorhandene Viren inaktiviert.

Gute Ergebnisse im Hinblick auf den Dehydratisierungsgrad werden dabei insbesondere erhalten, wenn die Dermis in dem Verfahrensschritt e) mehrmals mit einem organischen Lösungsmittel behandelt wird. Vorzugsweise wird die Dermis zwei- bis zehnmal, besonders bevorzugt vier- bis achtmal und ganz besonders bevorzugt sechsmal mit organischem Lösungsmittel behandelt. Um anfangs einen langsamen Wasserentzug bzw. eine schonende Dehydratisierung zu erreichen, ist es zudem bevorzugt, in dem ersten Schritt ein mit Wasser verdünntes organisches Lösungsmittel, beispielsweise eine 70 %-ige Acetonlösung, einzusetzen, bevor dann in den folgenden Behandlungsschritten höherkonzentrierte verdünnte organische Lösungsmittel, wie beispielsweise bei dem zweiten Behandlungsschritt eine 90 %-ige Acetonlösung, und danach konzentriertes organisches Lösungsmittel, wie unverdünntes Aceton, eingesetzt werden.

Dabei beträgt die Dauer der einzelnen Behandlungsschritte in dem Verfahrensschritt e) vorzugsweise 1 bis 48 Stunden, wobei zumindest einer dieser Behandlungsschritte bevorzugt für 5 bis 48 Stunden, besonders bevorzugt für 12 bis 36 Stunden und höchst bevorzugt für 20 bis 30 Stunden, beispielsweise für 24 Stunden, durchgeführt wird.

Vorzugsweise wird auch der Verfahrensschritt e) in der zuvor für den Verfahrensschritt b) als bevorzugt beschriebenen rotierenden Trommel durchgeführt.

Nach Abschluss der Dehydratisierung wird restliches organisches Lösungsmittel in der Dermis vorzugsweise durch eine Umlufttrocknung bei 25 bis 60°C, beispielsweise bei 35°C, entfernt.

Wie dargelegt ist das vorliegende Verfahren insbesondere zur Herstellung eines Transplantats aus porciner Dermis geeignet. Allerdings eignet sich dieses auch für alle andere tierischen Dermis, wie beispielsweise Rinderdermis.

Gemäß einer weiteren besonders bevorzugten Ausführungsform der vorliegenden Erfindung umfasst der Verfahrensschritt a) insbesondere in dem Fall porciner Dermis die nachfolgenden Schritte:
a₁) Reinigen der Haut von geschlachtetem Tier, insbesondere Schwein, mit ggf. Detergens enthaltendem Wasser, wobei das Wasser eine Temperatur zwischen Raumtemperatur und maximal 40°C aufweist,
a₂) Abziehen der Haut von der Rückenpartie des Schweins,
a₃) Entfernen der Borsten von der in dem Schritt a₂) bereitgestellten Haut und
a₄) Entfernen der Subcutis und der Epidermis von der Dermis mittels einer Spaltmaschine.

Im Rahmen der vorliegenden Erfindung wurde herausgefunden, dass neben der Art der chemischen Behandlung auch die Art der Vorbereitung der tierischen Dermis einen wichtigen Einfluss auf die Qualität des erhaltenen Transplantats ausübt. Insbesondere wurde im Rahmen der vorliegenden Erfindung festgestellt, dass durch ein herkömmliches Verfahren in einem Schlachthof hergestellte Schweinehaut zu Transplantaten mit vergleichsweise schlechten Eigenschaften führt. Dies liegt daran, dass bei den herkömmliches Schlachthofverfahren die Schweine nach der Schlachtung mit etwa 65°C heißem Wasser gebrüht werden, um diese oberflächlich zu reinigen und zu desinfizieren, bevor anschließend die Borsten abgeflammt werden. Durch diese thermische Behandlung wird das in der Schweinehaut enthaltene Kollagen irreversibel geschädigt bzw. denaturiert.

Um dies zu vermeiden, ist es gemäß der vorstehenden, besonders bevorzugten Ausführungsform der vorliegenden Erfindung vorgesehen, die Haut von geschlachtetem Schwein schonend zu reinigen, nämlich mit maximal 40°C warmen Wasser, welches ggf. mildes Detergens enthält. Mit anderen Worten wird bei dem erfindungsgemäßen Verfahren keine thermische Behandlung der Haut mit mehr als 40°C heißem Medium durchgeführt.

Vorzugsweise wird in dem erfindungsgemäßen Verfahren nur der Hautabschnitt von der Rückenpartie des Tieres, vorzugsweise Schweins, verwendet, weil sich diese im Vergleich zu der Haut der Bauchseite durch eine straffere Hautstruktur und eine größere Dicke auszeichnet. Mithin werden in dem Verfahrensschritt a₂) von der kompletten Haut die Anteile der Bauchseite entfernt und verworfen und nur die Haut der Rückenpartie mit einer Fläche von etwa 800 x 1200 mm verwendet.

Anschließend werden in dem Verfahrensschritt a₃) vorzugsweise die Borsten von der Haut mechanisch, und zwar bevorzugt mit einer Klinge, entfernt, bevor die Haut in beispielsweise etwa 400 x 600 mm große Stücke geschnitten wird. Die Hautdicke beträgt in diesem Stadium etwa 2,5 bis 3 mm. Die so bereitgestellte Haut kann direkt dem Verfahrensschritt a₄) zugeführt werden oder zunächst bei einer Temperatur von maximal -20°C gelagert werden.

Danach werden in dem Verfahrensschritt a₄) von der Dermis die Subcutis und die Epidermis vorzugsweise mittels einer Spaltmaschine entfernt. Zu diesem Zweck enthält die Bandmessermaschine vorzugsweise ein Endlosmesser, welches mit einem Bandsägeblatt ohne Zähne vergleichbar ist, und welches über zwei Räder angetrieben und über zusätzliche Rollen geführt wird. Vorzugsweise ist das Messer horizontal gelagert und wird mit einer Geschwindigkeit von mehreren Metern/Sekunde bewegt. Mit einer solchen Spaltmaschine ist die Spaltdicke stufenlos einstellbar. Dabei wird die Dermis dem Messer durch zwei Walzen zugeführt und entlang der Querschnittsfläche in zwei Stücke geteilt.

In Weiterbildung des Erfindungsgedankens wird es vorgeschlagen, in dem Verfahrensschritt a₄) zunächst die Subcutis von der Dermis zu entfernen, bevor anschließend die Epidermis von der Dermis entfernt wird. Für den ersten Teilschritt der Entfernung der Subcutis von der Dermis wird die Maschine beispielsweise auf ein Spaltmaß von 2 mm eingestellt. Die vorgeschnittenen Hautstücke werden dann mit der Fettseite nach oben in die Maschine eingeführt, wobei der über eine Dicke von 2 mm hinausgehende Fett- und Bindegewebeanteil inklusive der Haarwurzeln abgetrennt und dann verworfen wird. Alternativ dazu kann das Spaltmaß auch auf etwa 1,5 oder etwa 1,0 mm eingestellt werden.

In dem nächsten Teilschritt der Entfernung der Epidermis von der Dermis wird das Spaltmaß der Spaltmaschine auf 0,5 mm eingestellt und die in dem vorherigen Teilschritt erhaltene Haut wird erneut in die Spaltmaschine eingeführt, um die Epidermis abzuspalten und zu verwerfen.

Die so bereitgestellte Dermis kann direkt dem Verfahrensschritt b) zugeführt werden oder zunächst bei einer Temperatur von maximal -20°C oder alternativ dazu bei 4°C in 25 %-iger Natriumchloridlösung gelagert werden.

Nach der Durchführung der Vorbehandlung und der chemischen Behandlung der Dermis gemäß den Verfahrensschritten a) bis d) wird schließlich aus der in dem Schritt e) dehydratisierten Dermis das Transplantat ggf. unter Verwendung einer Schablone ausgeschnitten und/oder ausgestanzt. Dabei können die Transplantate zu rechteckigem, rundem oder ovalem Formen geschnitten werden. Optional dazu können die Transplantate auch mit einem Lochmuster gestanzt werden.

Anschließend werden die Transplantate vorzugsweise doppelt in Flachbeutel aus laminierter Kunststofffolie bzw. Verbundfolie aus Kunststoff und Aluminiumfolie eingeschweißt. Dabei werden die Transplantate vorzugweise trocken verpackt. Allerdings können die Transplantate nach einer erfolgten Rehydratisierung auch feucht verpackt werden.

Zur Sterilisation können die verpackten Transplantate schließlich mit Gammastrahlung behandelt werden, beispielsweise mit Gammastrahlung mit einer Energiedosis von 17 bis 22 kGy. Während trockene Transplantate vorzugsweise bei einer Temperatur von 20 bis 40°C bestrahlt werden, wird die Bestrahlung von feuchten Transplantaten bevorzugt bei einer Temperatur von -20 bis -80°C durchgeführt.

Ein weiterer Gegenstand der vorliegenden Erfindung ist ein mit dem erfindungsgemäßen Verfahren erhältliches Transplantat.

Beispielsweise kann das erfindungsgemäße Transplantat eine rechteckige Form mit einer Größe von beispielsweise 50 x 100 mm bis 350 x 350 mm, eine runde Form mit einem Durchmesser zwischen 30 und 100 mm oder eine ovale Form mit einer Größe von beispielsweise 100 x 155 mm bis 250 x 350 mm aufweisen.

Ferner betrifft die vorliegende Erfindung die Verwendung des zuvor beschriebenen Transplantats in der Hernienchirurgie, beispielsweise zur Behandlung von Narbenhernien oder zur Behandlung von Platzbauch.

Bei der Behandlung von Hernien wird das erfindungsgemäße Transplantat mit dem umliegenden intakten Gewebe vernäht und dient zunächst als Defektverschluss. Im Rahmen des sich anschließenden Gewebemodellings dient das Transplantat als Leitschiene für patienteneigene Zellen, welche die Kollagenstruktur des Transplantats besiedeln. Durch den Umbau des Transplantats, d.h. den Abbau des Transplantatkollagens und den parallelen Aufbau von patienteneigenem Gewebe, wird der Defekt durch neu gebildetes Bindegewebe geschlossen.

Nachfolgend wird die vorliegende Erfindung anhand eines diese nicht beschränkenden Ausführungsbeispiels näher erläutert.

### Beispiel

Ein frisch geschlachtetes Schwein wurde oberflächlich mit 40°C warmen Wasser, welches 0,1 bis 1 Gew.-% Natriumdodecylsulfat (SDS) enthielt, gereinigt, bevor dieses gehäutet wurde. Von der kompletten Haut wurden die Anteile der Bauchseite entfernt und verworfen und es wurde nur die Haut der Rückenpartie mit einer Fläche von etwa 800 x 1200 mm weiterverarbeitet.

Anschließend wurden von der Schweinehaut die Borsten mit einer Klinge mechanisch entfernt, bevor die Haut in etwa 400 x 600 mm große Stücke geschnitten wurde. Danach wurde die Haut mit der Fettseite nach oben in eine auf ein Spaltmaß von 2 mm eingestellte Spaltmaschine eingeführt, um den über eine Dicke von 2 mm hinausgehende Fett- und Bindemittelanteile inklusive der Haarwurzeln abzutrennen. Dabei enthielt die Spaltmaschine ein über zwei Räder angetriebenes und über zusätzliche Rollen geführtes, horizontal gelagertes Endlosmesser.

Im Anschluss daran wurde das Spaltmaß der Spaltmaschine auf 0,5 mm eingestellt und die Haut wurde erneut durch die Spaltmaschine geführt, um die Epidermis abzuspalten. So wurde eine Dermis mit einer Dicke von 1,5 mm erhalten.

Danach wurde die Dermis gemäß dem nachfolgenden Behandlungsschema in einer rotierenden Trommel behandelt:

| **Schritt** | **Aktion** | **Zeit (h)** | **Zweck** |
|---|---|---|---|
| 1 | Spülen mit Wasser (3x) | | |
| 2 | Lagern in Wasser | 0,5 | |
| 3 | Lagern in Wasser | 0,5 | |
| 4 | Behandeln mit wässriger 1% Natriumsulfid, 0,1 M NaOH-Lösung | 16 | Haarentfernung |
| 5 | Spülen mit Wasser (3x) | | Diffusion, Osmose |
| 6 | Lagern in Wasser | 1 | |
| 7 | Lagern in 10% NaCl | 3 | |
| 8 | Lagern in Wasser | 1 | |
| 9 | Lagern in 10% NaCl | 16 | |
| 10 | Spülen mit Wasser (3x) | | Diffusion, Osmose |
| 11 | Lagern in Wasser | 1 | |
| 12 | Lagern in 10% NaCl | 3 | |
| 13 | Lagern in Wasser | 1 | |
| 14 | Lagern in 10% NaCl | 16 | |
| 15 | Spülen mit Wasser (3x) | | |
| 16 | Lagern in 3% Wasserstoffperoxid | 24 | Inaktivierung von Bakterien und Viren, Bleichen von Pigmenten |
| 17 | Lagern in 3% Wasserstoffperoxid | 24 | |
| 18 | Lagern in 3% Wasserstoffperoxid | 24 | |
| 19 | Spülen mit Wasser (3x) | | Auswaschen H₂O₂ |
| 20 | Lagern in 70 % Aceton | 2 | |
| 21 | Lagern in 90 % Aceton | 2 | langsamer Wasserentzug |
| 22 | Lagern in 100 % Aceton | 2 | Wasserentzug/ Entfettung |
| 23 | Lagern in 100 % Aceton | 16 | Wasserentzug/ Entfettung |
| 24 | Lagern in 100 % Aceton | 24 | Wasserentzug/ Entfettung |
| 25 | Lagern in 100 % Aceton | 24 | Vireninaktivierung |
| 26 | Trocknung bei 35°C | 24 | Acetonentzug |

Nach der chemischen Behandlung wurden schließlich aus der Dermis ovale Transplantate mit einer Größe von jeweils 160 x 250 mm ausgestanzt und diese wurden paarweise in einer laminierten Kunststofffolie trocken eingeschweißt sowie bei 35°C mit Gammastrahlung mit einer Energiedosis von 20 kGy bestrahlt.

Die so hergestellten Transplantate wiesen eine der nativen Struktur nahezu identische Morphologie auf.

## Patentansprüche

1. Verfahren zur Herstellung eines Transplantats aus tierischer Dermis, insbesondere aus porciner Dermis, umfassend die nachfolgenden Schritte:
a) Bereitstellen von tierischer Dermis,
b) Behandeln der tierischen Dermis mit einer wässrigen, alkalischen Natriumsulfid enthaltenden Lösung, welche 0,01 bis 1,5 Gew.-% Natriumsulfid enthält,
c) ein- oder mehrmaliges Behandeln der Dermis mit einer wässrigen Salzlösung,
d) ein- oder mehrmaliges Behandeln der Dermis mit einer wässrigen Wasserstoffperoxidlösung und
e) Dehydratisieren der Dermis.

2. Verfahren nach Anspruch 1,
**dadurch gekennzeichnet, dass**
in dem Schritt b) eine wässrige Lösung eingesetzt wird, welche 0,75 bis 1,5 Gew.-% und bevorzugt 1 Gew.-% Natriumsulfid enthält.

3. Verfahren nach Anspruch 1 oder 2,
**dadurch gekennzeichnet, dass**
in dem Schritt b) eine wässrige Lösung eingesetzt wird, welche neben Natriumsulfid 0,001 bis 0,5 M Natriumhydroxid, bevorzugt 0,005 bis 0,4 M Natriumhydroxid, besonders bevorzugt 0,01 bis 0,3 M Natriumhydroxid, insbesondere bevorzugt 0,05 bis 0,2 M Natriumhydroxid, ganz besonders bevorzugt 0,075 bis 0,125 M Natriumhydroxid und höchst bevorzugt 1 M Natriumhydroxid enthält.

4. Verfahren nach zumindest einem der vorstehenden Ansprüche,
**dadurch gekennzeichnet, dass**
die Dermis in dem Schritt b) für 1 bis 48 Stunden, bevorzugt für 5 bis 36 Stunden, besonders bevorzugt für 12 bis 24 Stunden und ganz besonders bevorzugt für 14 bis 20 Stunden mit einem, bezogen auf das der Dermis, 5- bis 15-fachen Volumen, bevorzugt 6- bis 14-fachen Volumen, insbesondere bevorzugt 7- bis 13-fachen Volumen, weiter bevorzugt 8- bis 12-fachen Volumen und ganz besonders bevorzugt 9- bis 11-fachen Volumen einer wässrigen Natriumsulfid enthaltenden Lösung behandelt wird.

5. Verfahren nach zumindest einem der vorstehenden Ansprüche,
**dadurch gekennzeichnet, dass**
die Dermis in dem Schritt c) mit einer wässrigen, 1 bis 50 Gew.-%, bevorzugt 2 bis 20 Gew.-%, besonders bevorzugt 5 bis 15 Gew.-% und ganz besonders bevorzugt 10 Gew.-% Salz enthaltenden Lösung behandelt wird, wobei das Salz bevorzugt ein Alkalimetallhalogenid oder Erdalkalimetallhalogenid, besonders bevorzugt ein Alkalimetallchlorid oder Erdalkalimetallchlorid, wie Kaliumchlorid, Lithiumchlorid, Magnesiumchlorid oder Calciumchlorid, ist.

6. Verfahren nach zumindest einem der vorstehenden Ansprüche,
**dadurch gekennzeichnet, dass**
die Dermis in dem Schritt c) zwei- bis zehnmal, bevorzugt drei- bis fünfmal und besonders bevorzugt viermal mit einer wässrigen Salzlösung behandelt wird, wobei die Dermis zwischen den Behandlungsschritten mit Salzlösung jeweils mit Wasser behandelt und/oder gespült wird.

7. Verfahren nach zumindest einem der vorstehenden Ansprüche,
**dadurch gekennzeichnet, dass**
die Dermis in dem Schritt d) mit einer wässrigen Lösung behandelt wird, welche 1 bis 10 Gew.-% Wasserstoffperoxid, bevorzugt 2 bis 5 Gew.-% Wasserstoffperoxid und besonders bevorzugt 3 Gew.-% Wasserstoffperoxid enthält.

8. Verfahren nach zumindest einem der vorstehenden Ansprüche,
**dadurch gekennzeichnet, dass**
die Dermis in dem Schritt d) zwei- bis fünfmal und bevorzugt dreimal mit einer wässrigen Wasserstoffperoxidlösung behandelt wird, wobei die Dermis zwischen den Behandlungsschritten mit der Wasserstoffperoxidlösung jeweils mit Wasser behandelt und/oder gespült wird.

9. Verfahren nach zumindest einem der vorstehenden Ansprüche,
**dadurch gekennzeichnet, dass**
die Dermis in dem Schritt e) durch zwei- bis zehnmaliges, bevorzugt durch vier- bis achtmaliges und besonders bevorzugt durch sechsmaliges Behandeln mit einem organischen Lösungsmittel ausgewählt aus der Gruppe bestehen aus Aceton, Methanol, Ethanol und Isopropylalkohol dehydratisiert wird.

10. Verfahren nach zumindest einem der vorstehenden Ansprüche,
**dadurch gekennzeichnet, dass**
in dem Schritt a) porcine Dermis bereitgestellt wird.

11. Verfahren nach Anspruch 10,
**dadurch gekennzeichnet, dass**
das Bereitstellen der Dermis in dem Schritt a) die folgenden Schritte umfasst:
a₁) Reinigen der Haut von geschlachtetem Schwein mit ggf. Detergens enthaltendem Wasser, wobei das Wasser eine Temperatur zwischen Raumtemperatur und maximal 40°C aufweist,
a₂) Abziehen der Haut von der Rückenpartie des Schweins,
a₃) Entfernen der Borsten von der in dem Schritt a₂) bereitgestellten Haut und
a₄) Entfernen der Subcutis und der Epidermis von der Dermis mittels einer Spaltmaschine.

12. Transplantat erhältlich nach einem Verfahren nach zumindest einem der vorstehenden Ansprüche.

13. Transplantat nach Anspruch 12 zur Verwendung in der Hernienchirurgie, zur Behandlung von Narbenhernien oder zur Behandlung von Platzbauch.

## Claims

1. A method for producing a transplant from animal dermis, in particular from porcine dermis, comprising the following steps:
a) providing animal dermis;
b) treating the animal dermis with an aqueous alkaline solution containing sodium sulfide, with 0.01 to 1.5% by weight sodium sulfide being contained;
c) treating the dermis once or several times with an aqueous saline solution;
d) treating the dermis once or several times with an aqueous hydrogen peroxide solution; and
e) dehydrating the dermis.

2. A method in accordance with claim 1,
**characterized in that**
an aqueous solution is used in step b) which contains 0.75 to 1.5% by weight sodium sulfide and preferably 1% by weight sodium sulfide.

3. A method in accordance with claim 1 or claim 2,
**characterized in that**
an aqueous solution is used in step b) which contains, in addition to sodium sulfide, 0.001 to 0.5 M sodium hydroxide, preferably 0.005 to 0.4 M sodium hydroxide, particularly preferably 0.01 to 0.3 M sodium hydroxide, in particular preferably 0.5 to 0.2 M sodium hydroxide, very particularly preferably 0.075 to 0.125 M sodium hydroxide and most preferably 1 M sodium hydroxide.

4. A method in accordance with at least one of the preceding claims,
**characterized in that**
the dermis is treated in step b) for 1 to 48 hours, preferably for 5 to 36 hours, particularly preferably for 12 to 24 hours, and very particularly preferably for 14 to 20 hours with a volume of an aqueous solution containing sodium sulfide relative to the dermis of 5-fold to 15-fold, preferably a volume of 6-fold to 14-fold, in particular preferably a volume of 7-fold to 13-fold, further preferably a volume of 8-fold to 12-fold, and very particularly preferably a volume of 9-fold to 11-fold.

5. A method in accordance with at least one of the preceding claims,
**characterized in that**
the dermis is treated in step c) with an aqueous solution containing 1 to 50% by weight salt, preferably 2 to 20% by weight salt, particularly preferably 5 to 15% by weight salt, and very particularly preferably 10% by weight salt, with the salt preferably being an alkaline metal halide or an earth alkaline metal halide, particularly preferably an alkaline metal chloride or an earth alkaline metal chloride such as potassium chloride, lithium chloride, magnesium chloride or calcium chloride.

6. A method in accordance with at least one of the preceding claims,
**characterized in that**
the dermis is treated two to ten times, preferably three to five times and particularly preferably four times with an aqueous saline solution in step c), with the dermis being treated and/or rinsed with water in each case between the treatment steps with saline solution.

7. A method in accordance with at least one of the preceding claims,
**characterized in that**
the dermis is treated with an aqueous solution in step d) which contains 1 to 10% by weight hydrogen peroxide, preferably 2 to 5% by weight hydrogen peroxide, and particularly preferably 3% by weight hydrogen peroxide.

8. A method in accordance with at least one of the preceding claims,
**characterized in that**
the dermis is treated two to five times, and preferably three times, with an aqueous hydrogen peroxide solution in step d), with the dermis being treated and/or rinsed with water in each case between the treatment steps with the hydrogen peroxide solution.

9. A method in accordance with at least one of the preceding claims,
**characterized in that**
the dermis is dehydrated in step e) by two-time to ten-time treatment, preferably by four-time to eight-time treatment, and particularly preferably by six-time treatment with an organic solvent selected from the group comprising acetone, methanol, ethanol and isopropyl alcohol.

10. A method in accordance with at least one of the preceding claims,
**characterized in that**
porcine dermis is provided in step a).

11. A method in accordance with claim 10,
**characterized in that**
the provision of the dermis in step a) comprises the following steps:
a₁) cleansing the skin of a slaughtered pig using water optionally containing detergent, with the water having a temperature between room temperature and a maximum of 40°C;
a₂) removing the skin from the back area of the pig;
a₃) removing the bristles from the skin prepared in step a₂); and
a₄) removing the subcutis and the epidermis from the dermis using a cutting machine.

12. A transplant which can be obtained in accordance with a method in accordance with at least one of the preceding claims.

13. A transplant in accordance with claim 12 for use in hernia surgery, for treating incisional hernias or for treating a burst abdomen.

## Revendications

1. Procédé de production d'un greffon à partir d'un derme animal, en particulier d'un derme de proc, comprenant les étapes suivantes de :
a) mise à disposition d'un derme animal,
b) traitement du derme animal avec une solution aqueuse contenant du sulfure de sodium alcalin, qui contient 0,01 à 1,5 % en poids de sulfure de sodium,
c) traitement une ou plusieurs fois du derme avec une solution saline aqueuse,
d) traitement une ou plusieurs fois du derme avec une solution aqueuse de peroxyde d'hydrogène et
e) déshydratation du derme.

2. Procédé selon la revendication 1, **caractérisé en ce que** l'on met en oeuvre à l'étape b) une solution aqueuse qui contient 0,75 à 1,5 % en poids et de préférence 1 % en poids de sulfure de sodium.

3. Procédé selon la revendication 1 ou 2, **caractérisée en ce que** l'on met en oeuvre à l'étape b) une solution aqueuse qui contient, en plus du sulfure de sodium, 0,001 à 0,5 M d'hydroxyde de sodium, de préférence 0,005 à 0,4 M d'hydroxyde de sodium, particulièrement préférentiellement 0,01 à 0,3 M d'hydroxyde de sodium, notamment préférentiellement 0,05 à 0,2 M d'hydroxyde de sodium, tout particulièrement préférentiellement 0,075 à 0,125 M d'hydroxyde de sodium, et le plus préférentiellement 1 M d'hydroxyde de sodium.

4. Procédé selon au moins une des revendications précédentes, **caractérisé en ce que** le derme à l'étape b) est traité pendant 1 à 48 heures, de préférence 5 à 36 heures, particulièrement préférentiellement 12 à 24 heures et tout particulièrement préférentiellement 14 à 20 heures, avec un volume de 5 à 15 fois, de préférence 6 à 14 fois, particulièrement préférentiellement 7 à 13 fois, plus préférentiellement 8 à 12 fois et tout particulièrement préférentiellement 9 à 11 fois d'une solution aqueuse contenant du sulfure de sodium, par rapport à celui du derme.

5. Procédé selon au moins une des revendications précédentes, **caractérisé en ce que** le derme à l'étape c) est traité avec une solution aqueuse contenant 1 à 50 % en poids, de préférence 2 à 20 % en poids, particulièrement préférentiellement 5 à 15 % en poids, et tout particulièrement préférentiellement 10 % en poids de sel, le sel étant de préférence un halogénure de métal alcalin ou un halogénure de métal alcalino-terreux, particulièrement préférentiellement un chlorure de métal alcalin ou un chlorure de métal alcalino-terreux, comme le chlorure de potassium, le chlorure de lithium, le chlorure de magnésium ou le chlorure de calcium.

6. Procédé selon au moins une des revendications précédentes, **caractérisé en ce que** le derme à l'étape c) est traité deux à dix fois, de préférence trois à cinq fois et particulièrement préférentiellement quatre fois avec une solution aqueuse de sel, le derme étant traité et/ou rincé avec de l'eau à chaque fois entre les étapes de traitement avec la solution de sel.

7. Procédé selon au moins une des revendications précédentes, **caractérisé en ce que** le derme à l'étape d) est traité avec une solution aqueuse qui contient 1 à 10 % en poids de peroxyde d'hydrogène, de préférence 2 à 5 % en poids de peroxyde d'hydrogène et particulièrement préférentiellement 3 % en poids de peroxyde d'hydrogène.

8. Procédé selon au moins une des revendications précédentes, **caractérisé en ce que** le derme à l'étape d) est traité et/ou rincé avec de l'eau deux à cinq fois et de préférence trois fois, le derme étant traité et/ou rincé avec de l'eau à chaque fois entre les étapes de traitement avec la solution de peroxyde d'hydrogène.

9. Procédé selon au moins une des revendications précédentes, **caractérisé en ce que** le derme à l'étape e) est traité par deux à dix traitements, de préférence quatre à huit traitements et particulièrement préférentiellement par six traitements avec un solvant organique choisi dans le groupe constitué par l'acétone, le méthanol, l'éthanol et l'alcool isopropylique.

10. Procédé selon au moins une des revendications précédentes, **caractérisé en ce que** le derme de porc est mis à disposition à l'étape a).

11. Procédé selon la revendication 10, **caractérisé en ce que** la mise à disposition du derme à l'étape a) comprend les étapes suivantes de :
a₁) nettoyage de la peau de porc abattu avec de l'eau contenant éventuellement des détergents, l'eau présentant une température située entre la température ambiante et au maximum 40 °C,
a₂) arrachage de la peau de la partie dorsale du porc,
a₃) retrait des poils de la peau mise à disposition à l'étape a₂) et
a₄) retrait de l'hypoderme et de l'épiderme du derme au moyen d'une machine de séparation.

12. Greffon pouvant être obtenu d'après un procédé selon au moins une des revendications précédentes.

13. Greffon selon la revendication 12 pour une utilisation dans la chirurgie des hernies, pour le traitement des hernies de cicatrice ou pour le traitement d'une déhiscence d'une plaie opératoire.
